# EUROPEAN PATENT SPECIFICATION

(11) **EP 2 497 833 B1**
(45) Date of publication and mention of the grant of the patent: **11.04.2018**
(21) Application number: 10827869.8
(22) Date of filing: 22.10.2010
(51) Int. Cl.: C12P 21/02, C12P 19/04

(54) **METHOD FOR PREPARING YEAST MANNOSE PROTEIN PRODUCT**
VERFAHREN ZUR HERSTELLUNG EINES HEFE-MANNOSE-PROTEINPRODUKTS
PROCÉDÉ DE PRÉPARATION D'UN PRODUIT À BASE DE PROTÉINES DE MANNOSE DE LEVURE

(30) Priority: 06.11.2009 CN 200910212346
(43) Date of publication of application: 12.09.2012
(73) Proprietor: Angel Yeast Co., Ltd., Hubei 443003 (CN)
(72) Inventor: YU, Xuefeng, Yichang Hubei 443003 (CN); LI, Zhihong, Yichang Hubei 443003 (CN); YU, Minghua, Yichang Hubei 443003 (CN); YAO, Juan, Yichang Hubei 443003 (CN); ZHANG, Yan, Yichang Hubei 443003 (CN); ZHANG, Haibo, Yichang Hubei 443003 (CN)
(74) Representative: Long, Giorgio
(86) International application number: PCT/CN2010/077991
(87) International publication number: WO 2011/054255

(56) References cited:
- EP-A2- 0 695 801
- WO-A2-2008/128973
- WO-A2-2008/128973
- CN-A- 1 162 333
- CN-A- 1 940 084
- CN-A- 101 087 872
- CN-A- 101 117 358
- CN-A- 101 570 769
- SAEKI K ET AL: "Purification and characterization of an alkaline protease from Oerskovia xanthineolytica TK-1", JOURNAL OF FERMENTATION AND BIOENGINEERING, SOCIETY OF FERMENTATION TECHNOLOGY, JP, vol. 77, no. 5, 1 January 1994 (1994-01-01), pages 554-556, XP025752788, ISSN: 0922-338X, DOI: 10.1016/0922-338X(94)90128-7 [retrieved on 1994-01-01]
- DATABASE MEDLINE [Online] US NATIONAL LIBRARY OF MEDICINE (NLM), BETHESDA, MD, US; 1977, VRSANSKÁ M ET AL: "Enzymes of the yeast lytic system produced by Arthrobacter GJM-1 bacterium and their role in the lysis of yeast cell walls.", XP002708021, Database accession no. NLM337691 & VRSANSKÁ M ET AL: "Enzymes of the yeast lytic system produced by Arthrobacter GJM-1 bacterium and their role in the lysis of yeast cell walls.", ZEITSCHRIFT FÜR ALLGEMEINE MIKROBIOLOGIE 1977, vol. 17, no. 6, 1977, pages 465-480, ISSN: 0044-2208
- CAMERON ET AL: "The Mannoprotein of Saccharomyces cerevisiae is an effective Bioemulsifier", APPLIED AND ENVIRONMENTAL MICROBIOLOGY, AMERICAN SOCIETY FOR MICROBIOLOGY, US, vol. 54, no. 6, 1 June 1988 (1988-06-01), pages 1420-1425, XP002087061, ISSN: 0099-2240
- CONWAY J ET AL: "THE EFFECT OF THE ADDITION OF PROTEASES AND GLUCANASES DURING YEAST AUTOLYSIS ON THE PRODUCTION AND PROPERTIES OF YEAST EXTRACTS", CANADIAN JOURNAL OF MICROBIOLOGY, NRC RESEARCH PRESS, CA, vol. 47, no. 1, 1 January 2001 (2001-01-01), pages 18-24, XP009044657, ISSN: 0008-4166, DOI: 10.1139/CJM-47-1-18

## Description

### Technical field

The present invention relates to a method for preparing a yeast mannoprotein product.

### Background art

Mannoprotein is composed by covalently linking a mannose polymer to a protein skeletal structure, and situated on the outermost layer of the yeast wall. Mannoprotein is one the main components of yeast cell wall. The yeast mannoprotein has a molecular weight of 20-200kD, and is composed of 5%-20% peptide and 80%-95% mannose. The backbone chain of mannan is α -1,2- mannan, which is linked to serine, threonine or aspartic acid of the protein or peptide with an O-glycosidic bond or N-glycosidic bond.

Scientific research has shown that the mannoprotein favours the stabilization of protein and tartar in wine, can weaken astringency and acidulous taste of tannin in wine and can increase the taste of fullness and richness of wine. Chardonnay (dry white wine) produced in the Burgundy districts of France is usually fermented in oak barrels, and then aged in wine lees for several months, wherein it is stirred 1-2 times once a week during the process of aging. Researches have shown that tartar in wine would be unstable if wine and wine lees are separated in late March, whereas the stability of tartar in wine would be very good if wine and wine lees are separated in late July. This phenomenon was not researched and evidenced in theory until recent years. Wine brewed by the foregoing method is rich in cell wall polysaccharides, in which the main polysaccharide is mannoprotein. The main effects of applying mannoprotein in wine include the following aspects:
1) preventing precipitation of potassium tartrate;
2) synergistic effect with redolence;
3) promoting fermentation of malic acid-lactic acid;
4) synergistic effect with polyphenol substances to increase mellow, full and soft taste of wine and weaken astringency taste of tannin;
5) preventing sediment of protein.

Currently, there are mainly two methods for preparing yeast mannoprotein: heating extraction and enzyme extraction.

Pyrolysis: the method is common, and comprises heat-treating yeast buffer solution with pH of 7 at high temperature to obtain the product. In 1977, Herlin, prepared mannoprotein from active dry yeast according to the following method: washing active dry yeast with distilled water, preparing a suspension with buffer solution having a pH of 7, stewing the suspension in a pot for 90 min at a temperature of 125 °C (this process is operated twice), centrifuging, precipitating the centrifuged liquid with 3 times alcohol based on volume of the centrifuged liquid, and drying to obtain the mannoprotein.

Enzymolysis: the yeast get the cell wall is usually degraded with β -1,3-glucanase to get the product of yeast mannoprotein. Johanna Van Rinsum, et al., (Van RINSUM J, KLIS Fs M, Van Den ENDE H. Cell wall glucomannoproteins of Saccharomy cerevisiae [J]. Yeast, 1991(7):717-726) separate mannan from yeast cell wall with laminarinase, carry out purification with affinity chromatography and anion exchange chromatography and get mannan in which the molar ratio of N-acetyl glucosamine, mannose, and glucose is 1:53:4. Yuxiang Zhang (Yuxiang Zhang, Zhuorong Yin, Process for Purifying mannoprotein and Assaying the molecular weight thereof [J]. Yeast, Wine-brewing Science and Technology 2005(4):72-74) extracts mannoprotein from waste beer yeast with the method of enzymolysis, and the process comprises: waste beer yeast→ pretreatment→breaking wall homogeneously→autolysis→centrifugal separation→precipitating (cell wall) → enzymolysis→centrifugation→concentration of supernatant→spray drying→ product in form of powder. Polysaccharides content is 60.57% and protein content is 28.1% in the product. Patent document US6139891 discloses a method for preparing yeast mannoprotein, which comprises treating the cell wall with glucanase and then membrane separation to get yeast mannoprotein. Moreover it has been proved that the product has the effect of stabilizing tartar when used in wine. WO2006/121803 A1 discloses a method for preparing yeast mannoprotein, which includes carrying out autolysis of yaest, treating the cell wall with protease and membrane separation to get yeast mannoprotein and yeast dextran simultaneously.

EP 1094117A1 discloses a method for preparing mannoprotein product, which comprises: pyrolyzing the yeast cell wall in the condition of pH 6-7 and temperature of 98-100°C for 15-30h, centrifuging to collect supernatant, precipitating supernatant at least 24h at a temperature of 4°C to remove colloid by-product, centrifuging to collect supernatant, vacuum concentration, and spray drying to obtain foregoing product. Patent application CN 1940084A discloses a method for preparing yeast mannoprotein, which comprises: collection of wine lees yeast, enzymolysis, heat treatment, precipitation with alcohol and obtaining yeast mannoprotein product.

Plenty of protein is extracted simultaneously during the heat-treatment process of extracting yeast mannoprotein, which makes difficult post-treatment and reduces extraction efficiency (Zhaohui Hu etc, The Progress of Study in mannoprotein obtained from Saccharomyces cerevisiae [J]. 34. (4): 64-66). Yeast mannoprotein obtained by treating with an enzyme also contains a certain amount of protein, and the yield is lower because the structure of the cell wall is not loose enough when only using an enzymatic treatment. Besides, the appropriate molecular weight of yeast mannoprotein is significant on whether yeast mannoprotein can bring enough effect in wine, so yeast mannoprotein obtained without using membrane separation not only influences its effect, but also brings a bad effect to wine because of massive protein.

### Summary of the invention

Raw material used in the present invention is the cell wall of *Saccharomyces cerevisiae* or cell walls of yeast belonging to other *Saccharomyces.* The raw material can be purchased from the market or obtained from *Saccharomyces cerevisiae* or yeast belonging to other *Saccharomyces* species by means of autolysis and separation. The method of the present invention is defined in claim 1 an comprises:
1) treating the yeast cell wall at high temperature and high pH value the pH value of the yeast cell wall is adjusted to 7.0-10.0 with inorganic acids such as dilute hydrochloric acid, sulfuric acid, and phosphoric acid, etc., and the yeast cell wall is heat treated by heating at a temperature of 80-135°C
2) treating with protease: the temperature of the yeast cell wall treated in step 1) is adjusted to 30-70°C, alkaline protease is added to treat the yeast cell wall, and the suspension is centrifuged to collect a supernatant;
3) precipitating at low temperature: the supernatant obtained in step 2) is cooled with ice water to a low temperature of 0-20°C in a storage tank, kept at such temperature for 1-20h, and centrifuged to remove precipitation;
4) membrane separating: the supernatant obtained in step 3) is membrane separated with a membrane having molecular weight cut-off (MWCo) of 200-400 kD preferably 400 kD wherein the apparatus used for membrane separating can be various tubular or spiral membrane separating apparatus using an organic the filtrate membrane or inorganic membrane, and then the filtrate is collected and spray dried to obtain the yeast mannoprotein product, wherein the following step is further comprised in said step 3) adjusting the pH value of said supernatant to 3.0-6.0 before carrying out a vacuum -concentration.
Preferably, in said step 1), the concentration of the yeast cell wall is adjusted to 5-15wt% by adding deionized water.

Preferably, the yeast cell wall is heat-treated for 0.5-5h in said step 1).

Preferably, the concentration of the alkaline protease may be 0.01-0.5wt% in said step 2).

Preferably, the following step is further comprised in said step 2): heating the yeast cell wall treated with alkaline protease to a temperature of 90 °C so as to kill the enzyme. Preferably, the yeast cell wall is treated with protease for 2-15h in said step 2).

Preferably, the centrifugal force of the centrifugal separation is more than 5000g in said step 2). The vacuum concentration step of the supernatant of step 3) is performed with a multi-effect concentrator until the concentration of dry substance thereof is 10-40wt% before carrying out the precipitation at low temperature. In step 3): adjusting pH value of said supernatant to 3.0-6.0 is performed with inorganic acids such as dilute hydrochloric acid, sulfuric acid, and phosphoric acid, etc. before carrying out the vacuum-concentration.

The technical solution of extracting mannoprotein in the present invention is precisely opposite to the teachings of prior art, i.e., supernatant is precipitated at low temperature in acid condition. Moreover the purpose of the membrane separation in the present invention lies in making mannoprotein pass through, which is opposite to the process for intercepting mannoprotein by using the membrane separation in prior art. The clarity of filtrate is obviously higher than that of retention liquid in membrane separation. A large mass of high molecule weight of protein and other polysaccharides are also contained in retention liquid, which will bring adverse effect on the stabilization when the product is applied to wine. These protein and polysaccharides with high molecular weight are removed by intercepting, which facilitates yeast mannoprotein to bring the effect of stabilizing wine tartar and protein in wine. Meanwhile, the required time on pyrolysis in the present method is shorter than that in prior art, so that the experiment steps are simplified and experiment cost is reduced. Besides, tests are carried out several times for choosing parameters and improving the technical solution.

### Embodiment

### Example 1

The pH value of the cell wall of *Saccharomyces cerevisiae* (Fubang yeast cell wall, Angel Yeast Co., Ltd) purchased from market is adjusted to 7.0 with dilute hydrochloric acid, and then the adjusted cell wall is heated to a temperature of 100°C and kept for 5h. The temperature of the yeast cell wall is adjusted to 50°C, alkaline protease with percentage concentration of 0.01 wt% is added to treat the yeast cell wall for 15h, and then the obtained suspension is centrifuged to collect a supernatant.

The supernatant is cooled with ice water until its temperature is 0°C in storage tank, kept at the temperature for 10h, and centrifuged to remove precipitate.

The centrifuged supernatant is membrane separated with hollow fiber membranes having MWCo of 200kD and then the filtrate is collected and spray dried to obtain a yeast mannoprotein product.

The product is sample 1.

### Example 2

Deionized water is added to yeast cell wall until the percentage concentration of the yeast cell wall is adjusted to 5wt%, wherein the yeast cell wall is obtained from *Saccharomyces cerevisiae* by means of autolysis and separation. Dilute sulfuric acid is added until the pH value of the yeast cell wall is adjusted to 10.0, and then the adjusted yeast cell wall is heated up to a temperature of 80°C and kept for 5h. The temperature of the yeast cell wall is adjusted to 30°C, alkaline protease with percentage concentration of 0.5 wt% is added to treat the yeast cell wall for 8h, the treatment of heating to 90 °C is carried out to kill the enzyme, and then the obtained suspension is centrifuged to collect the supernatant. The pH value of the supernatant is adjusted to 3.0 with dilute sulfuric acid. The adjusted supernatant is vacuum-concentrated with multi-effect concentrator until the percentage concentration of dry substance thereof is 30wt%, cooled with ice water until its temperature is 20°C in the storage tank, kept at the temperature for 1h, and centrifuged to remove precipitation.

The centrifuged supernatant is membrane separated with ceramic membranes having MWCo of 200kD and then the filtrate is collected and spray dried to obtain a yeast mannoprotein product.

The product is sample 2.

### Example 3

Deionized water is added to the yeast cell wall until the percentage concentration of the yeast cell wall is adjusted to 15wt%, wherein the yeast cell wall is obtained from *Saccharomyces cerevisiae* by means of autolysis and separation. Dilute sulphuric acid is added until pH value of the yeast cell wall is adjusted to 8.0, and then the adjusted yeast cell wall is heated up to a temperature of 135°C and kept for 0.5h. The temperature of the yeast cell wall is adjusted to 50°C, alkaline protease with percentage concentration of 0.2 wt% is added to treat the yeast cell wall for 2h, the treatment of heating to 90°C is carried out to kill the enzyme, and then the obtained suspension is centrifuged while centrifugal force is 5000g to collect the supernatant. The pH value of the supernatant is adjusted to 6.0 with dilute phosphoric acid. The adjusted supernatant is vacuum-concentrated with a multi-effect concentrator until the percentage concentration of dry substance thereof is 10wt%, cooled with ice water until its temperature is 10°C in the storage tank, kept at the temperature for 20h, and centrifuged to remove precipitation.

The centrifuged supernatant is membrane separated with ceramic membranes having MWCo of 300 kD and then filtrate is collected and spray dried to obtain a yeast mannoprotein product.

The product is sample 3.

### Example 4

Deionized water is added to yeast cell wall until the percentage concentration of the yeast cell wall is adjusted to 10wt%, wherein the yeast cell wall is obtained from *Saccharomyces cerevisiae* by means until the of autolysis and separation. Dilute hydrochloric acid is added until the pH value of the yeast cell wall is adjusted to 8.0, and then the adjusted yeast cell wall is heated up to a temperature of 110°C and kept for 3h.

Temperature of the yeast cell wall is adjusted to 50°C, alkaline protease with percentage concentration of 0.2 wt% is added to treat the yeast cell wall for 10h, the treatment of heating to 90 °C is carried out to kill enzyme, and then obtained suspension is centrifuged while centrifugal force is 7000g to collect supernatant.

pH value of the supernatant is adjusted to 5.0 with dilute hydrochloric acid. The adjusted supernatant is vacuum concentrated with multi-effect concentrator until percentage concentration of dry substance thereof is 40wt%, cooled with ice water until its temperature is 10°C in storage tank, kept at the temperature for 10h, and centrifuged to remove precipitation.

The centrifuged supernatant is membrane separated with hollow fiber membranes having MWCo of 400 kD, and then filtrate is collected and spray dried to obtain yeast mannoprotein product.

The product is sample 4.

Table regarding the data of each component in the obtained samples such as mass percentage content and molecular weight is showed as follows:

| | Sample 1 | Sample 2 | Sample 3 | Sample 4 |
|---|---|---|---|---|
| Mannan content | 42% | 53% | 41% | 48% |
| Protein content | 19% | 14% | 21% | 16% |
| Molecular weight | 167893 | 174873 | 226775 | 298441 |

## Claims

1. A method for preparing a yeast mannoprotein product comprising the following steps:
1) adjusting the pH value of the yeast cell wall to 7.0-10.0, and heat-treating the yeast cell wall in a temperature range of 80-135°C;
2) adjusting the temperature of the yeast cell wall treated in step 1) to 30-70°C, adding an alkaline protease to treat the yeast cell wall, and centrifuging the obtained suspension to collect the supernatant
3) keeping the supernatant obtained in step 2) at a low temperature of 0-20°C for 1-20h and centrifuging it to remove the precipitate
4) membrane-separating the supernatant obtained in step 3) with a membrane having a molecular weight cut-off of 200-400 kD, and collecting and spray-drying the filtrate to obtain the yeast mannoprotein product; wherein, the following step is further comprised in said step 3): adjusting the pH value of said supernatant to 3.0-6.0 before carrying out a vacuum-concentration.

2. The method according to claim 1, **characterized in that** the concentration of the yeast cell wall is adjusted to 5-15wt% in said step 1).

3. The method according to claim 1, **characterized in that** the yeast cell wall is heat-treated for 0.5-5h in said step 1).

4. The method according to claim 1, **characterized in that** the concentration of the alkaline protease is 0.01-0.5wt% in said step 2).

5. The method according to claim 1, **characterized in that** the yeast cell wall is treated with the protease for 2-15h in said step 2).

6. The method according to claim 1, **characterized in that** the following step is further comprised in said step 2): heating the yeast cell wall treated with alkaline protease to a temperature of 90 °C so as to kill the enzyme.

7. The method according to claim 1, **characterized in that** the centrifugal force of the centrifugal separation is more than 5000g in said step 2).

8. The method according to claim 1, **characterized in that** the following step is further comprised in said step 3): vacuum-concentrating said supernatant until the concentration of dry substance thereof is 10-40wt% before carrying out the precipitation at low temperature.

9. The method according to claim 1, **characterized in that** the supernatant is separated with a membrane having a molecular weight cut-off of 400 kD in said step 4).

## Patentansprüche

1. Verfahren zum Herstellen eines Hefe-Mannoprotein-Produkts, umfassend die folgenden Schritte:
1) Anpassen des pH Werts der Hefe-Zellwand auf 7,0 - 10,0 und Wärmebehandeln der Hefe-Zellwand in einem Temperaturbereich von 80 - 135 °C;
2) Anpassen der Temperatur der in Schritt 1) behandelten Hefe-Zellwand auf 30 - - 70 °C, Zugeben einer alkalischen Protease um die Hefe-Zellwand zu behandeln und Zentrifugieren der erhaltenen Suspension um den Überstand zu sammeln
3) Halten des in Schritt 2) erhaltenen Überstands bei einer niedrigen Temperatur von 0 - 20 °C für 1 - 20 h und ihn Zentrifugieren, um den Niederschlag zu entfernen
4) Membranabtrennung des in Schritt 3) erhaltenen Überstands mit einer Membran, die eine Molekulargewicht-Ausschlussgrenze von 200 - 400 kD aufweist, und Sammeln und Sprühtrocknen des Filtrats, um das Hefe-Mannoprotein-Produkt zu erhalten;
wobei
der folgende Schritt fernen in dem Schritt 3) umfasst ist: Anpassen des pH Werts des Überstands auf 3,0 - 6,0 bevor eine Vakuum-Aufkonzentration durchgeführt wird.

2. Verfahren nach Anspruch 1, **dadurch gekennzeichnet, dass** die Konzentration der Hefezellwand in dem Schritt 1) auf 5 - 15 Gewichts-% angepasst wird.

3. Verfahren nach Anspruch 1, **dadurch gekennzeichnet, dass** die Hefezellwand in dem Schritt 1) für 0,5 - 5 h wärmebehandelt wird.

4. Verfahren nach Anspruch 1, **dadurch gekennzeichnet, dass** die Konzentration der alkalischen Protease in dem Schritt 2) 0,01 - 0,5 Gewichts-% ist.

5. Verfahren nach Anspruch 1, **dadurch gekennzeichnet, dass** die Hefezellwand in dem Schritt 2) für 2 - 15 h mit der Protease behandelt wird.

6. Verfahren nach Anspruch 1, **dadurch gekennzeichnet, dass** der folgende Schritt fernen in dem Schritt 2) umfasst ist: Erwärmen der Hefezellwand, die mit alkalischer Protease behandelt ist, auf eine Temperatur von 90 °C um das Enzym abzutöten.

7. Verfahren nach Anspruch 1, **dadurch gekennzeichnet, dass** die Zentrifugalkraft der Zentrifugalabtrennung in dem Schritt 2) mehr als 5000 g ist.

8. Verfahren nach Anspruch 1, **dadurch gekennzeichnet, dass** der folgende Schritt fernen in dem Schritt 3) umfasst ist: Vakuum-Aufkonzentration des Überstands bis die Konzentration an trockener Substanz davon 10 - 40 Gewichts-% ist, bevor die Fällung bei niedriger Temperatur durchgeführt wird.

9. Verfahren nach Anspruch 1, **dadurch gekennzeichnet, dass** der Überstand in dem Schritt 4) mit einer Membran abgetrennt wird, die eine Molekulargewicht-Ausschlussgrenze von 400 kD aufweist.

## Revendications

1. Procédé de préparation d'un produit à base de mannoprotéine de levure, comprenant les étapes suivantes :
(1) ajustement de la valeur de pH de la paroi cellulaire de la levure entre 7,0 et 10,0, et thermotraitement de la paroi cellulaire de la levure à une plage de températures de 80 à 135 °C ;
2) ajustement de la température de la paroi cellulaire de la levure traitée à l'étape 1) entre 30 et 70 °C, ajout d'une protéase alcaline pour traiter la paroi cellulaire de la levure, et centrifugation de la suspension obtenue pour collecter le surnageant ;
3) conservation du surnageant obtenu à l'étape 2) à une faible température comprise entre 0 et 20 °C pendant 1 à 20 heures et centrifugation de celui-ci pour retirer le précipité ;
4) Séparation par membrane du surnageant obtenu à l'étape 3) avec une membrane présentant une coupure de poids moléculaire de 200 à 400 kD, et récupération et séchage par pulvérisation du produit de filtration pour obtenir le produit de mannoprotéine de levure,
dans lequel l'étape suivante est en outre comprise dans ladite étape 3) : ajustement de la valeur de pH dudit surnageant entre 3,0 et 6,0 avant de réaliser une concentration sous vide.

2. Procédé selon la revendication 1, **caractérisé en ce que** la concentration de la paroi cellulaire de la levure est ajustée entre 5 et 15 % en poids dans ladite étape 1).

3. Procédé selon la revendication 1, **caractérisé en ce que** la paroi cellulaire de la levure est thermotraitée pendant 0,5 à 5 heures dans ladite étape 1).

4. Procédé selon la revendication 1, **caractérisé en ce que** la concentration de la protéase alcaline est comprise entre 0,01 et 0,5 % en poids dans ladite étape 2.

5. Procédé selon la revendication 1, **caractérisé en ce que** la paroi cellulaire de la levure est traitée avec la protéase pendant 2 à 15 h dans ladite étape 2.

6. Procédé selon la revendication 1, **caractérisé en ce que** l'étape suivante est en outre comprise dans ladite étape 2) : chauffage de la paroi cellulaire de la levure traitée avec la protéase alcaline à une température de 90 °C de façon à tuer l'enzyme.

7. Procédé selon la revendication 1, **caractérisé en ce que** la force centrifuge de la séparation centrifuge est supérieure à 5 000 g dans ladite étape 2).

8. Procédé selon la revendication 1, **caractérisé en ce que** l'étape suivante est en outre comprise dans ladite étape 3) : concentration sous vide dudit surnageant jusqu'à ce que la concentration de sa substance sèche se trouve entre 10 à 40 % en poids avant de réaliser la précipitation à faible température.

9. Procédé selon la revendication 1, **caractérisé en ce que** le surnageant est séparé avec une membrane ayant une coupure de poids moléculaire de 400 kD dans ladite étape 4).
